# EUROPÄISCHE PATENTSCHRIFT

(11) **EP 3 308 805 B1**
(45) Veröffentlichungstag und Bekanntmachung des Hinweises auf die Patenterteilung: **11.12.2024**
(21) Anmeldenummer: 17194332.7
(22) Anmeldetag: 02.10.2017
(51) Int. Cl.: A61L 2/18, A61L 2/24, G01N 17/00, G01N 27/00, A61L 2/28

(54) **REINIGUNGSÜBERWACHUNG MIT BELAGSSENSOREN**
CLEANING MONITORING WITH COATING SENSORS
SURVEILLANCE DE NETTOYAGE AU MOYEN DES CAPTEURS DE REVÊTEMENT

(30) Priorität: 13.10.2016 DE 102016219964
(43) Veröffentlichungstag der Anmeldung: 18.04.2018
(73) Patentinhaber: KRONES Aktiengesellschaft, 93073 Neutraubling (DE)
(72) Erfinder: Habersetzer, Florian, 93073 Neutraubling (DE); Soellner-Wein, Gertrud, 93073 Neutraubling (DE); Beierle, Eva, 93073 Neutraubling (DE); Oehmichen, Thomas, 73093 Neutraubling (DE); Engelhard, Patrick, 93073 Neutraubling (DE); Feilner, Roland, 93073 Neutraubling (DE); Piana, Stefan, 93073 Neutraubling (DE)
(74) Vertreter: Grünecker Patent- und Rechtsanwälte PartG mbB

(56) Entgegenhaltungen:
- WO-A1-2009/135504
- WO-A1-2016/122351
- WO-A1-2016/171629
- JP-A- 2014 197 029
- RU-C1- 2 452 927
- US-A1- 2013 003 048
- TECHNOPARKSTRASSE: "greenTEG AG", 4 August 2016 (2016-08-04), XP055469102, Retrieved from the Internet <URL:https://shop.greenteg.com/wp-content/uploads/gSKIN_HeatFlux_Datasheet_v3.6.pdf> [retrieved on 20180420]

## Beschreibung

### Stand der Technik

Die Erfindung betrifft ein System der im Oberbegriff des Patentanspruchs 1 angegebenen Art und ein Verfahren der im Oberbegriff des Patentanspruchs 9 angegebenen Art.

Die Reinigung von Anlagen bzw. Prozessanlagen, z.B. Anlagen zur Lebensmittelproduktion, stellt einen essenziellen Schritt für die Produktsicherheit dar. Herkömmlicherweise werden Anlagen daher nach vorgegebenen Reinigungsprogrammen zu festgelegten Zeitpunkten gereinigt.

Zur Feststellung des Verschmutzungsgrades bzw. des Reinigungsgrades einer Anlage werden dabei insbesondere kapazitative Sensoren zur Messung von Belagbildung eingesetzt.

So beschreibt z.B. die EP 1 376 112 B1 einen kapazitiven Sensor, welcher eine Belagbildung über einen speziell ausgeformten Kondensator misst, dessen Wirkungsweise darauf beruht, dass die elektrische Impedanz des Kondensators von den dielektrischen Eigenschaften des sich auf dem Kondensator ablagernden Belags abhängt. Weitere bekannte Mittel und Verfahren zur Detektion von Ablagerungen auf Bauteilen von Anlagen sind in der WO 2009/135507 A1 und in der US 2013/003048 A1 beschrieben. Ein beispielhaftes Verfahren zur Kalibrierung von thermoelektrischen Sensoren wird in der WO 2016/122351 A1 beschrieben.

Nachteilig an der bekannten Sensorik und Methodik zur Feststellung des Verschmutzungsgrades bzw. des Reinigungsgrades einer Anlage bzw. Lebensmittelanlage ist unter anderem, dass der Belagsschichtaufbau nur für begrenzte Belagsschichtdicken gemessen werden kann und beispielsweise ein effektives Produktions- und Reinigungszyklus übergreifendes Monitoring des Hygienezustandes der Anlage nicht möglich ist.

Dies kann unter anderem zu ineffektiven und ineffizienten Reinigungsvorgängen und verkürzter Produktionszeit führen, da in Abwesenheit genauerer Daten über den Hygienezustandes der Anlage und durch angewendete überdimensionierte Sicherheitszuschläge unnötig lange Reinigungszeiten entstehen können, sowie ein damit einhergehend unnötiger Verbrauch an Ressourcen, wie z.B. Wasser, Chemikalien und Energie, erfolgt.

Auch können so fehlterminierte, d.h. zu früh oder zu spät angesetzte, Reinigungsprozesse die Folge sein, da keine genaue Prüfung der Notwendigkeit einer Reinigung möglich ist.

Unter einem Reinigungsprozess können dabei beispielsweise alle aus dem Stand der Technik bekannten Verfahren bzw. Verfahrensabläufe verstanden werden, um eine Lebensmittelanlage ohne wesentliche Demontage frei von jeglichen Verunreinigungen zu bekommen. Insbesondere zählen hierzu beispielsweise die sogenannten "Cleaning in Place"- bzw. CIP- als auch die sogenannten "Sterilisation in Place" bzw. SIP-Verfahren. CIP-Verfahren können dabei wie folgt ablaufen: Spülen der Anlage mit Wasser, Reinigen der Anlage mit einem alkalischen Mittel, z.B. bei erhöhter Temperatur, Spülen der Anlage, Reinigen der Anlage mit einem sauren Reinigungsmittel, z.B. bei erhöhter Temperatur, Spülen der Anlage mit Wasser. Soll z.B. zusätzlich sterilisiert werden (SIP), kann die Anlage mit einem Sterilisationsmittel wie z.B. Wasser mit einer Temperatur von größer 120°C oder einem chemischen Sterilisationsmittel behandelt werden. Je nach Anforderung kann auch nur eine basische Reinigung ausreichend sein. Bei besonders schwer zu reinigenden Produkten kann auch vor der basischen Reinigung zusätzlich mit Säure gereinigt werden.

### Aufgabe

Es ist somit Aufgabe der Erfindung Mittel bereitzustellen mit denen die Überwachung des Hygienezustandes einer Anlage bzw. Prozessanlage, z.B. einer Anlage zur Lebensmittelproduktion, verbessert werden kann, insbesondere beispielsweise hinsichtlich der Effizienz und Wirksamkeit der Überwachung des Hygienezustandes, sowie hinsichtlich der Effizienz und Wirksamkeit der Reinigung der Anlage.

### Lösung

Dies wird erfindungsgemäß durch ein System nach Anspruch 1 und ein Verfahren nach Anspruch 9 erreicht. Vorteilhafte Ausführungsformen und Weiterbildungen sind Gegenstand der Unteransprüche.

Ein System zum Überwachen des Hygienezustandes einer Prozessanlage, z.B. Lebensmittelanlage, und zum Überwachen und Steuern eines Reinigungsprozesses der Prozessanlage, z.B. Lebensmittelanlage, umfasst wenigstens zwei Belagssensoren zur Messung von Belägen auf Teilen der Prozessanlage, z.B. Lebensmittelanlage, umfassen, wobei die wenigstens zwei Belagssensoren verschiedene Messbereiche aufweisen. Ferner können die wenigstens zwei Belagssensoren dazu konfiguriert sein, nach verschiedenen Messprinzipien arbeiten zu können.

Unter Belägen können dabei jedwede unerwünschte Ablagerungen auf Anlagenteilen, insbesondere z.B. auf Anlageninnenwänden (insbesondere z.B. Innenwandungen von Rohrleitungen), wie beispielsweise unter anderem mineralische, organische und gemischt organischmineralische Ablagerungen, verstanden werden.

Typische Beläge bzw. Verunreinigungen, wie mineralische Substanzen, Proteine oder Stärke können sich dabei insbesondere auf Teilen bzw. Oberflächen mit gegenüber der Umgebung erhöhter Temperatur, wie z.B. auf Wärmetauschern, ablagern.

Unter der Messung von Belägen sind Messungen der Belagsschichtdicke zu verstehen.

Die Messung von Belägen kann jedoch auch die Messung von anderen physikalischen Eigenschaften des Belags und/oder der Umgebung der Prozessanlage, z.B. Lebensmittelanlage, an denen sich der Belag bildet und/oder eines Produktes der Prozessanlage, z.B. Lebensmittelanlage, beispielsweise eines durch die Prozessanlage, z.B. Lebensmittelanlage, durchströmenden Produktes oder Reinigungsmediums, umfassen.

Unter einer zu messenden Eigenschaft bzw. unter einer zu messenden physikalischen Eigenschaft kann dabei beispielsweise eine elektrische oder thermische Leitfähigkeit, eine elektrische Kapazität, eine Wärmekapazität, und/oder optische Absorptions- und/oder Emissionseigenschaften, z.B. Fluoreszenzeigenschaften, und/oder eine oder mehrere der folgenden Eigenschaften wie Strömungsgeschwindigkeit, Durchflussgeschwindigkeit, Trübung, Farbe, pH-Wert, Temperatur, Druck bzw. Druckverlust in der Anlage, Wärmedurchgangswert einer Rohraußenwand der Anlage, Konzentration bestimmter Partikel im Reinigungsmedium und/oder im Produkt, Anwesenheit flüchtiger Komponenten; Produkteigenschaften des bearbeiteten Produkts, beispielsweise Viskosität, Zusammensetzung, Messwerte von Online-Titrationen, Transmission- oder Reflexionsmessungen, Messwerte von Reaktionen aus verschiedenen Online-Fliess-Injektions-Analysen, UV-Adsorptionsmesswerte, Schwingungs- und Dämpfungseigenschaften, Ultraschallmessungen, wellenlängenabhängige Lichtstreuung, und/oder optische Aktivität (Polarisationswinkeländerung), verstanden werden.

Die Verwendung von Belagssensoren, die verschiedene Messbereiche aufweisen und/oder die nach unterschiedlichen Messprinzipien arbeiten können, kann eine sichere, vollständigere und kontinuierliche Überwachung des Hygienezustandes der Anlage über Produktions- und Reinigungszyklen hinweg erlauben. Insbesondere können der Verschmutzungsgrad der Anlage während der Produktion und der Reinigungsgrad bzw. der Abreinigungsgrad während eines Reinigungsprozesses der Anlage besser überwacht und gesteuert werden.

Anstelle einer Reinigung nach starr vorgegebenen Parametern oder z.B. nur in Abhängigkeit eines einzelnen unzuverlässigen nur eine begrenzte Belagsschichtdicke messenden kapazitiven Belagssensors, kann es ein erfindungsgemäßes System ermöglichen, die Anlage je nach Bedarf zu reinigen.

Mit anderen Worten kann der Zeitpunkt, an dem eine Reinigung sinnvoll oder nötig ist, und/oder der Zeitpunkt an dem die Reinigung beendet werden kann, besser bestimmt werden und beispielsweise vermieden werden, unnötige Reinigungen durchzuführen, welche unnötige Kosten und unnötigen Energieaufwand verursachen.

Auch die Effizienz und Effektivität mit der ein Reinigungsprozess überwacht werden kann, kann dadurch erhöht werden. Dies kann unter anderem zur Minimierung von Stillstandzeiten und Minimierung des Verbrauchs an Ressourcen, wie beispielsweise Reinigungsmedien, führen.

Durch erfindungsgemäß ermöglichtes Reinigen der Anlage nach Notwendigkeit in Abhängigkeit der Verschmutzung mit fortschreitender Produktion sowie ein exakt auf die Verschmutzung anpassbares Reinigungsprogramm, kann also die zur Reinigung benötigte Zeit auf ein Minimum reduziert werden und gleichzeitig die Produktsicherheit erhöht werden.

Beispielhafterweise kann das System dabei so gestaltet sein, dass wenigstens ein Belagssensor dazu konfiguriert sein kann, nahezu unbegrenzte Belagsschichtdicken zu messen, bzw. dazu konfiguriert sein kann, dickere bzw. stärkere Belagsschichtdicken messen zu können als z.B. im Vergleich zu einem kapazitiven Belagssensor. Beispielhafterweise kann ein derartiger Belagssensor ein Ultraschall-Belagssensor sein.

Beispielhafterweise kann das System dabei so gestaltet sein, dass wenigstens ein Belagssensor ein kapazitiver Belagssensor sein kann und wenigstens ein Belagssensor ein Ultraschall-Belagssensor sein kann.

Auch ist denkbar, dass alternativ oder zusätzlich wenigstens ein Belagssensor ein optischer Belagssensor oder ein thermischer Belagssensor oder ein Belagssensor mit Konduktionsmessung oder ein Belagssensor mit Fluoreszenzmessung oder ein Belagssensor mit Absorptionsmessung sein kann. Auch diese Art der Belagssensoren können dazu konfiguriert sein, nahezu unbegrenzte Belagsschichtdicken zu messen, bzw. dazu konfiguriert sein kann, dickere bzw. stärkere Belagsschichtdicken messen zu können, als beispielsweise im Vergleich zu einem kapazitiven Belagssensor.

Ein thermischer Belagssensor kann dabei beispielsweise einen Temperatursensor und wenigstens einen Temperaturemitter konfiguriert zur Einkopplung von Wärmeenergie, z.B. zur Beaufschlagung eines zeitlich veränderlichen Temperaturprofiles, in eine zu untersuchende Stelle der Prozessanlage, z.B. Lebensmittelanlage, umfassen.

Die Vielfalt der einsetzbaren Belagssensoren kann dabei die Genauigkeit, mit der eine Belagsbildung gemessen werden kann, erhöhen.

Ferner können sich die Messbereiche der Belagssensoren ergänzen. Darunter kann beispielsweise verstanden werden, dass die Messbereiche verschiedene Belagssensoren komplementär zueinander sind, wobei sich die Messbereiche teilweise überlappen können. Es ist jedoch auch möglich, dass sich die Messbereiche der verschiedenen Belagssensoren nicht überlappen. Ebenso ist jedoch denkbar, dass ein Messbereich des einen Belagssensors auch den Messbereich des/eines anderen enthält.

Beispielhafterweise kann der Messbereich wenigstens eines Belagssensors Belagschichtdicken von nahe oder gleich Null umfassen und/oder der Messbereich wenigstens eines weitere Belagssensors Belagschichtdicken mit einer maximal tolerierbaren Belagsschichtdicke umfassen.

Mit anderen Worten kann z.B. ein Belagssensor dünne Belagsschichtdicken messen und ein weiterer Belagssensor dicke Belagsschichtdicken messen. Dabei können beispielsweise unter dünnen Belagsschichtdicken, Belagsschichtdicken von 0 bis 0,5 mm, und unter dicken Belagsschichtdicken, z.B. Belagsschichtdicken von 0,5 mm bis 5 mm, und unter einer maximal tolerierbaren Belagsschichtdicke, z.B. Belagsschichtdicken bis 5 mm, verstanden werden.

Besagte beispielhafte Belagsschichtdicken bzw. Belagsschichtdickenwerte können dabei jedoch zudem von der Belagsart bzw. von der Belagshärte abhängig sein, da beispielsweise weichere Beläge leichter entfernbar sein können als härtere Beläge, können gegebenenfalls beispielsweise weichere Beläge eine größere maximal tolerierbaren Belagsschichtdicke aufweisen als vergleichsweise härtere Beläge.

Beispielsweise ist also denkbar, dass zwei verschiedene Belagssensoren verschiedene Belagsschichtdicken messen, beispielsweise ein kapazitiver Belagssensor dünne Belagsschichten bzw. dünne Belagsschichtdicken misst, und z.B. ein zweiter Belagssensor mittels eines Ultraschallmessverfahrens für das Messen von dickeren Belagsschichten bzw. dickerer Belagsschichtdicken eingesetzt wird.

Dies kann beispielsweise die Bestimmung erleichtern, ob und wann eine Reinigung der Prozessanlage, z.B. Lebensmittelanlage, nötig ist, beispielsweise bei einer Feststellung des Überschreitens einer maximal tolerierbaren Belagsschichtdicke durch den Belagssensor dessen Messbereich die maximal tolerierbaren Belagsschichtdicke umfasst.

Darüber hinaus ist denkbar, dass verschiedene Belagssensoren Messungen mit verschiedenen Auflösungen durchführen können. Beispielsweise wäre denkbar, dass ein Belagssensor für dünne Belagsschichtdicken mit einer höheren räumliche Auflösung bzw. Schichtdickenauflösung ausgestattet ist bzw. betrieben werden kann, als ein Belagssensor für dicke Belagsschichtdicken.

Dies kann beispielsweise eine genauere Überwachung des/eines Reinigungsverlaufes der Prozessanlage, z.B. Lebensmittelanlage, ermöglichen, da genauer festgestellt werden kann, ob und wann eine Verminderung der Belagsschichtdicke durch den Reinigungsprozess die Belagsschichtdicke auf Null oder auf nahezu Null innerhalb einer vorgegebenen Toleranz erreicht ist, und somit der zu reinigende Teil der bzw. die ganze Prozessanlage, z.B. Lebensmittelanlage, als sauber und hygienisch rein anzusehen ist.

Typische Auflösungen für die Messung von Belagschichtdicken können je nach Belagsart und Belagssensorart für dünne Beläge beispielsweise zwischen +/- 0,5 und +/- 5 µm liegen und für dickere Beläge zwischen +/- 5 und +/- 50 µm liegen.

Wie bereits erwähnt, kann wenigstens ein Belagssensor neben Messungen einer Belagsschichtdicke auch konfiguriert sein zur Messung einer anderen / von weiteren physikalischen Eigenschaft/en des zu untersuchenden Belages und/oder der Umgebung der Prozessanlage, z.B. Lebensmittelanlage an denen sich der Belag bildet und/oder eines Produktes der Prozessanlage, z.B. Lebensmittelanlage.

So kann ein Belagssensor z.B. konfiguriert sein zur Messung einer physikalischen Eigenschaft eines die zu untersuchende Stelle durchströmenden Mediums, insbesondere beispielsweise konfiguriert sein zur Messung der elektrischen Leitfähigkeit eines die zu untersuchende Stelle durchströmenden Mediums bzw. Fluides oder Fluidgemisches, beispielweise mittels elektrisch kapazitiver Messung von Änderungen in der elektrischen Leitfähigkeit des die zu untersuchende Stelle durchströmenden Mediums.

Die kann es beispielsweise erlauben den Reinigungsverlauf besser zu überwachen, da beispielsweise das Ausspülen eines Reinigungsmediums mit Wasser besser beobachtet und zeitlich geregelt werden kann.

In einem System zum Überwachen des Hygienezustandes einer Prozessanlage, z.B. Lebensmittelanlage, und zum Überwachen und Steuern eines Reinigungsprozesses der Prozessanlage, z.B. Lebensmittelanlage, kann dabei wenigstens ein Belagssensor, beispielsweise insbesondere wenigstens ein Belagssensor der konfiguriert ist, dünne Belagsschichtdicken messen zu können, an einer schwer zu reinigenden Stelle oder an der am schwersten zu reinigenden Stelle der Prozessanlage, z.B. Lebensmittelanlage, angebracht sein.

Beispielhafte Stellen für schwer bzw. am schwersten zu reinigende Stellen der Prozessanlage, z.B. Lebensmittelanlage, können z.B. Hochtemperaturbereiche von Wärmetauschern, Vordenaturierungsstufen, Ringkessel oder Zentralkessel einer Befüllungsvorrichtung, Füllventile, Puffertanks, Totstellen, Hinterschneidungen, Rohrerweiterungen, Übergänge von Dichtungen zu Materialen, beheizte Oberflächen, Oberflächen zur Wärmeübertragung bzw. mit einer Temperaturdifferenz, umfassen.

Darüber hinaus kann zusätzlich oder alternativ z.B. wenigstens ein Belagssensor, beispielsweise insbesondere wenigstens ein Belagssensor der konfiguriert ist, dickere bzw. stärkere Belagsschichtdicken messen zu können, auch an einer Stelle mit im Vergleich zu anderen Stellen der Prozessanlage, z.B. Lebensmittelanlage, verstärkter Belagsbildung, z.B. an einer Stelle mit maximaler Belagsbildung, angebracht sein. Die verstärkte Belagsbildung kann dabei beispielsweise durch eine an besagter Stelle herrschende die Belagsbildung fördernde Temperaturdifferenz an der Stelle im Vergleich zu umgebenden Stellen verursacht werden.

Diese Stelle mit vermehrter Belagsbildung kann dabei verschieden sein von einer schwer zu reinigenden Stelle, d.h. vergleichsweise leicht zu reinigen sein.

Eine derartige beispielhafte Anordnung kann z.B. helfen sicherzustellen, dass die Prozessanlage, z.B. Lebensmittelanlage, zuverlässig hygienisch gereinigt wurde, da man beispielsweise davon ausgehen kann, dass bei Feststellung einer zufriedenstellenden Reinigung einer schwer bzw. am schwersten zu reinigenden Stelle und/oder bei Feststellung einer zufriedenstellenden Reinigung einer im Vergleich zu anderen Stellen der Prozessanlage, z.B. Lebensmittelanlage, besonders stark verschmutzten Stelle der Prozessanlage, z.B. Lebensmittelanlage, die gesamte Prozessanlage, z.B. Lebensmittelanlage, vollständig und zufriedenstellend gereinigt wurde.

Somit kann insbesondere auch bei sehr starker Verschmutzung der Prozessanlage, z.B. Lebensmittelanlage, besser sichergestellt werden dass die Anlage zufriedenstellend hygienisch gereinigt wurde.

Darüber hinaus kann der wenigstens eine Belagssensor beheizbar sein. Damit kann beispielsweise eine Stelle der Prozessanlage, z.B. Lebensmittelanlage, simuliert werden, welche eine gegenüber anderen Teilen der Anlage erhöhte Temperatur aufweist, und somit anfälliger für verstärkte Belagsbildung sein kann.

Auf diese Weise kann beispielsweise auch für den Fall, dass der Belagssensor nicht direkt an eine derartige Stelle der Prozessanlage, z.B. Lebensmittelanlage, mit erhöhter Temperatur angebracht werden kann, sicher gestellt werden, dass die Belagsbildung bzw. der Hygienezustand und/oder Reinigungsverlauf für die Stelle der Prozessanlage, z.B. Lebensmittelanlage, mit erhöhter Temperatur überwacht und geregelt werden kann.

Ein Verfahren zum Überwachen des Hygienezustandes einer Prozessanlage, z.B. Lebensmittelanlage, und zum Überwachen und Steuern eines Reinigungsprozesses der Prozessanlage, z.B. Lebensmittelanlage, umfasst folgende Schritte:
- Eine Überwachung des Hygienezustandes bei Produktion durch Überwachen der Belagsbildung an wenigstens einem Teil der Prozessanlage, z.B. Lebensmittelanlage, während der Produktion, mittels wenigstens einem Belagssensors dessen Messbereich Belagsschichtdicken mit einer vorgegebenen maximal tolerierbaren Belagsschichtdicke umfasst,
- Eine Einleitung oder Initiierung eines Reinigungsprozesses der Prozessanlage, z.B. Lebensmittelanlage, bei Feststellung durch den wenigstens einen Belagssensor, dass die Belagsschichtdicke die vorgegebene maximal tolerierbare Belagsschichtdicke überschreitet bzw. überschritten hat,
- Überwachung des Hygienezustandes bei Produktion und/oder Überwachung und/oder Beendigung des eingeleiteten Reinigungsprozesses durch wenigstens einen weiteren Belagssensor, dessen Messbereich verschieden von dem Messbereich des ersten Belagssensor ist , insbesondere dessen Messbereich z.B. Belagschichtdicken von nahe oder gleich Null umfassen kann.

So kann beispielsweise eine bedarfsweise flexible Überwachung des Hygienezustandes der Prozessanlage, z.B. Lebensmittelanlage, bei Produktion und bei einem Reinigungsprozess gewährleistet werden, sowie die Zeitpunkte für Beginn und Beendigung einer/der Reinigung besser bestimmt werden, und so der Beginn und das Ende der Reinigung gezielt und genau gesteuert werden.

Dies kann beispielsweise die Genauigkeit mit der ein Reinigungsprozess überwacht werden kann verbessern, da insbesondere z.B. ein Reinigungsende besser und genauer festgestellt werden kann.

Auch ist denkbar, dass der zweite Belagssensor dessen Messbereich z.B. Belagschichtdicken von nahe oder gleich Null umfassen kann eine höhere Auflösung als der Belagssensor dessen Messbereich Belagsschichtdicken mit einer vorgegebenen maximal tolerierbaren Belagsschichtdicke umfasst, haben kann. Dies kann die Genauigkeit mit der ein Reinigungsprozess überwacht und z.B. ein Reinigungsende festgestellt werden kann, noch weiter steigern.

Dabei kann der wenigstens eine Belagssensor dessen Messbereich Belagsschichtdicken mit einer vorgegebenen maximal tolerierbaren Belagsschichtdicke umfassen kann, und/oder der optionale wenigstens eine Belagssensor dessen Messbereich Belagschichtdicken von nahe oder gleich Null umfasst, beispielsweise ein Belagssensor der oben beschriebenen Art sein, also beispielsweise ein kapazitiver Belagssensor, oder ein Ultraschall-Belagssensor, oder ein optischer Belagssensor, oder ein thermischer Belagssensor, oder ein Belagssensor mit Konduktionsmessung, oder ein Belagssensor mit Fluoreszenzmessung, oder ein Belagssensor mit Absorptionsmessung sein.

Ferner ist es beispielsweise möglich, dass das Überwachen der Belagsbildung an wenigstens einem Teil der Prozessanlage, z.B. Lebensmittelanlage, während der Produktion zunächst durch den Belagssensor erfolgt, dessen unterer Messbereich Belagschichtdicken von nahe oder gleich Null umfasst, und wobei erst bei Erreichen des oberen Endes des Messbereiches des Belagssensors mit unterem Messbereich von Belagschichtdicken von nahe oder gleich Null, die Überwachung des Hygienezustandes bei Produktion umgeschaltet wird auf den Belagssensors, dessen Messbereich Belagschichtdicken mit einer vorgegebenen maximal tolerierbaren Belagsschichtdicke umfasst.

Ebenso ist es beispielsweise möglich, dass die Überwachung des/eines eingeleiteten Reinigungsprozesses zunächst durch den/einen Belagssensor erfolgt, dessen unterer Messbereich Belagschichtdicken mit einer vorgegebenen maximal tolerierbaren Belagsschichtdicke umfasst, und wobei erst bei Erreichen des unteren Endes des Messbereiches des Belagssensor mit Messbereich für Belagschichtdicken mit einer vorgegebenen maximal tolerierbaren Belagsschichtdicke bzw. bei Erreichen seiner unteren Nachweisgrenze für Beläge bzw. Belagsschichtdicken, die Überwachung des eingeleiteten Reinigungsprozesses umgeschaltet wird auf den/einen Belagssensor, dessen unterer Messbereich Belagschichtdicken von nahe oder gleich Null umfasst, und der beispielsweise ein höhere Auflösung haben kann.

Wird beispielsweise auch die untere Nachweisgrenze oder eine vorgegebene Toleranzgrenze des Belagssensors, dessen unterer Messbereich Belagschichtdicken von nahe oder gleich Null umfasst, erreicht, kann beispielsweise der Reinigungsprozess auf Veranlassung des Belagssensors bzw. des Belagssensorsystems beendet werden. Hierzu kann beispielsweise zudem auch ein Signal ausgegeben werden, welches das Ende des Reinigungsprozess signalisieren kann.

Darüber hinaus kann die Überwachung und/oder Steuerung des/eines eingeleiteten Reinigungsprozesses zusätzlich die Überwachung eines Ausspülens, z.B. mit Wasser, eines Reinigungsmediums umfassen, wobei die Überwachung des Ausspülens mittels eines Sensors erfolgen kann, der dazu konfiguriert sein kann, eine physikalischen Eigenschaft eines die zu reinigende Stelle durchströmenden Mediums zu messen, also beispielsweise konfiguriert dazu sein kann, die elektrische Leitfähigkeit eines die zu reinigende Stelle durchströmenden Mediums zu messen.

Dies kann beispielsweise die Regelung des Reinigungsprozesses verbessern, sowie die Erkennung eines abgeschlossenen Reinigungsprozesses erleichtern.

Diese beispielhaften Verfahrensschritte können, wie erwähnt, die Hygienesicherheit und die Produktsicherheit verbessern, sowie die Produktionsstillstandzeit der Prozessanlage, z.B. Lebensmittelanlage, minimieren.

Wie erwähnt, kann ein für ein System zum Überwachen des Hygienezustandes einer Prozessanlage, z.B. Lebensmittelanlage, und zum Überwachen und Steuern eines Reinigungsprozesses einer Prozessanlage, z.B. Lebensmittelanlage, auch einen thermischen Belagssensor umfassen.

Dieser optionale beispielhafte thermische Belagssensor kann dabei wenigstens einen Temperatursensor und wenigstens einen Temperaturemitter umfassen, und konfiguriert sein zur Einkopplung von Wärmeenergie in eine, ggf. mit einem Belag verunreinigte, zu untersuchende Stelle der Prozessanlage, z.B. Lebensmittelanlage.

Beispielsweise kann der thermische Belagssensor konfiguriert sein zur Beaufschlagung eines zeitlich veränderlichen, z.B. periodisch veränderlichen, Temperaturprofiles in eine zu untersuchende Stelle der Prozessanlage, z.B. Lebensmittelanlage.

Die beispielsweise durch den Temperaturemitter an der zu untersuchenden Stelle / dem zu untersuchenden Bereich der Prozessanlage, z.B. Lebensmittelanlage, eingekoppelte Wärmeenergie bzw. das beaufschlagte zeitlich veränderliche Temperaturprofil, kann eine verzögerte Temperaturänderung bzw. eine verzögertes zeitlich veränderliches Temperaturprofil der zu untersuchenden Stelle der Anlage auslösen, deren Betrag bzw. Amplitude und Verzögerung bzw. Phase oder Phasenunterschied, beispielsweise vom Temperatursensor des thermischen Belagssensors gemessen werden kann. Aus diesen Messungen von Betrag bzw. Amplitude und Verzögerung (Phase bzw. Phasenverschiebung bzw. Phasenunterschied) der an der zu untersuchenden Stelle ausgelösten Temperaturänderung(en) können Rückschlüsse auf das Vorhandensein und die Belagsschichtdicke eines Belags gezogen werden.

Diese Art der Wärmeflussthermographie kann ebenfalls zu einer genaueren und verbesserten Überwachung des Verschmutzungsgrades der Anlage während der Produktion und der Überwachung des Reinigungsgrades bzw. des Abreinigungsgrades während eines Reinigungsprozesses der Anlage beitragen.

Der beispielhafte optional thermische Belagssensor bzw. ein beispielhafter Temperaturemitter des thermischen Belagssensors, umfassend z.B. Wärmestrahler und/oder Heiz- oder Kühlelement(e) und/oder Ultraschallsender, kann so konfiguriert sein:
- dass die Beaufschlagung eines zeitlich veränderlichen, z.B. periodisch veränderlichen, Temperaturprofiles in eine zu untersuchende Stelle der Prozessanlage, z.B. Lebensmittelanlage, durch periodisches Heizen und Kühlen eines Mediums, z.B. Reinigungsmediums, durch Heiz- bzw. Kühlelemente vor der zu untersuchende Stelle, beispielsweise bei konstantem Medienfluss, erfolgen kann, und/oder
- dass die Beaufschlagung eines zeitlich veränderlichen Temperaturprofiles in eine zu untersuchende Stelle der Prozessanlage, z.B. Lebensmittelanlage, durch Wärmebestrahlung von einer der zu untersuchenden Stelle gegenüberliegenden Stelle, der Einstrahlstelle, durch ein Medium, z.B. Produkt- oder Reinigungsmedium, hindurch erfolgen kann, wobei die die Einstrahlstelle beispielsweise so ausgelegt sein kann, dass dort eine verminderte Belagsbildung erfolgt bzw. vorhanden ist, wobei dies ggf. durch eine Kühlung der Einstrahlstelle, z.B. Peltierkühlung, erreicht werden kann, und/oder
- dass die Beaufschlagung eines zeitlich veränderlichen Temperaturprofiles in eine zu untersuchende Stelle der Prozessanlage, z.B. Lebensmittelanlage, durch Anregung der zu untersuchende Stelle mit Ultraschall durch ein Medium, z.B. Produkt- oder Reinigungsmedium hindurch erfolgen kann.

Mit anderen Worten kann alternativ oder zusätzlich ein Verfahren zum Überwachen des Hygienezustandes einer Prozessanlage, z.B. Lebensmittelanlage, und zum Überwachen und Steuern eines Reinigungsprozesses der Prozessanlage, z.B. Lebensmittelanlage, einen, einige oder alle der folgenden Schritte umfassen:
- eine Beaufschlagung eines zeitlich veränderlichen Temperaturprofiles auf ein zu untersuchendes Teil der Prozessanlage, z.B. Lebensmittelanlage,
- eine Messung der durch die Beaufschlagung an dem zu untersuchenden Teil der Prozessanlage, z.B. Lebensmittelanlage, ausgelösten Temperaturänderung mit Betrag und Verzögerung zur Ermittlung des Vorhandenseins und der Stärke der Belagsbildung, z.B. der Stärke der Belagsschichtdicke.

Hierzu kann insbesondere z.B. ein periodisches Temperaturprofil mit einer Frequenz im Bereich von 10 mHz bis 1 Hz beaufschlagt werden, bzw. mit einer experimentell ermittelten Frequenz in Abhängigkeit der mechanischen Dimensionierung der zu untersuchenden Stelle der Prozessanlage, z.B. Lebensmittelanlage.

Zudem kann beispielsweise die Temperaturverlaufsmessung der ausgelösten Temperaturänderung durch eine spezielle ausgestaltete zu untersuchende Stelle bzw. Messstelle erfolgen.

Hierzu ist beispielsweise denkbar, dass z.B. eine Außenwandung der Messstelle verjüngt und ggf. mechanisch verstärkt ist. Dies kann die Messung der ausgelösten Temperaturänderung vereinfachen ohne eine Beeinträchtigung der Homogenität der Messstelle zu verursachen.

Insbesondere kann dies ohne Beeinträchtigung der Homogenität einer Innenwandung der Messstelle erfolgen, so dass die Messstelle das gleiche Anlagerungsverhalten für Beläge wie die normalen Wandungsstellen aufweisen kann.

Die beschriebenen beispielhaften Schritte, können dabei folgende zwei physikalische Effekte ausnutzen.

Zum einen wird durch vorhandene Beläge an der zu untersuchenden Stelle eine Verzögerung, also eine Phasenverschiebung der Wärmeleitung zum Sensor, i.e. zum Temperatursensor des thermischen Belagssensors, erfolgen, welche mit der Belagsstärke zunimmt.

Zum anderen wird ein Belag (beispielhafter Emissionsgrad bei thermischer Infrarot-Strahlung ~ 0.9) den Wärmeeintrag bzw. die Wärmebeaufschlagung deutlich stärker aufnehmen als das Baumaterial der Anlage an der zu untersuchenden Stelle, beispielsweise blankpolierter Edelstahl (z.B. mit Emissionsgrad < 0.1), welches die einfallende Wärmestrahlung im Wesentlichen wegreflektiert, und damit wird vom Belag im Vergleich zu einem Anlagenteil ohne bzw. mit weniger Belag, die Amplitude des gemessen Temperaturverlaufsmessung der ausgelösten Temperaturänderung erhöht.

Der Vollständigkeit halber sei erwähnt, dass bei einer Reinigung der Anlage unter anderem sicherzustellen ist, dass die gesamte Oberfläche vollständig gereinigt wird. Je nach Komplexität der Bau- bzw. Anlagenteile sind die Oberflächen jedoch unterschiedlich schwer zu reinigen. Die Reinigung sollte daher beispielsweise so erfolgen, dass auch das ungünstigste Bauteil, welches am meisten verschmutzt bzw. am schwersten zu reinigen ist, vollständig gereinigt ist, so dass ein mögliches Hygienerisiko minimiert werden kann.

Die am schwersten zu reinigende Stelle ist meist durch eine komplexe mechanische Konstruktion gekennzeichnet, wie z.B. Hinterschneidungen, Übergänge von Dichtungen zu Materialien, Oberflächen zur Wärmeübertragung bzw. mit einer Temperaturdifferenz. Ihre Bestimmung kann sich unter anderem durch empirisches Ermitteln durch Reinigungsversuche, bzw. durch theoretische Bewertung der Konstruktion der Anlage unter Berücksichtigung der jeweiligen Umgebungs- bzw. Einsatzbedingungen, ergeben.

Daher kann alternativ oder zusätzlich ein Verfahren zum Überwachen des Hygienezustandes einer Prozessanlage, z.B. Lebensmittelanlage, und zum Überwachen und Steuern eines Reinigungsprozesses der Prozessanlage, z.B. Lebensmittelanlage, einen, einige oder alle der folgenden Schritte umfassen:
- eine direkte Messung des Verschmutzungsgrades oder Hygienezustandes oder Reinigungs- bzw. Abreinigungsgrades der Prozessanlage, z.B. Lebensmittelanlage, an einem Teil der Prozessanlage, z.B. Lebensmittelanlage, z.B. eine direkte Messung von Belägen an einem Teil der Prozessanlage, z.B. Lebensmittelanlage, wobei die Messung direkt an einer schwer zu reinigenden Stelle der Prozessanlage, z.B. Lebensmittelanlage, z.B. Hochtemperaturbereiche von Wärmetauschern, Vordenaturierungsstufen, Ringkessel oder Zentralkessel einer Befüllungsvorrichtung, Füllventile, Puffertanks, Totstellen, Hinterschneidungen, Rohrerweiterungen, Übergänge von Dichtungen zu Materialen, beheizte Oberflächen, Oberflächen zur Wärmeübertragung bzw. mit einer Temperaturdifferenz, erfolgen kann, und/oder
- eine Messung des Verschmutzungsgrades oder Hygienezustandes oder Reinigungs- bzw. Abreinigungsgrades der Prozessanlage, z.B. Lebensmittelanlage, über eine Messung des Verschmutzungsgrades oder Hygienezustandes an einem Indikatorbauteil, wobei das Indikatorbauteil eine schwer zu reinigende Stelle der Prozessanlage, z.B. Lebensmittelanlage, simulieren kann, und/oder das Indikatorbauteil eine Stelle der Prozessanlage, z.B. Lebensmittelanlage, mit verstärkter Belagsbildung simulieren kann.

Das Indikatorbauteil bzw. Referenzbauteil kann zur Verbesserung der Simulation einer schwer zu reinigenden Stelle, z.B. zur Simulation eines Wärmetauschers, bzw. zur Simulation einer Stelle mit verstärkter Belagsbildung, auch beheizt werden.

Die beschriebenen Schritte können die Effizienz und Effektivität der Überwachung des Hygienezustandes einer Prozessanlage, z.B. Lebensmittelanlage, und der Überwachung und Steuerung eines Reinigungsprozesses der Prozessanlage, z.B. Lebensmittelanlage, erhöhen, da beispielsweise davon ausgegangen werden kann, dass wenn die schwer bzw. die am schwierigsten zu reinigende Stelle bzw. die simulierte schwer zu reinigende Stelle sauber, d.h. z.B. ohne Belag ist, auch der Übrige Teil der Anlage sauber bzw. hygienisch rein ist.

Die Messung des Verschmutzungsgrades oder Hygienezustandes der Prozessanlage, z.B. Lebensmittelanlage, über eine Messung des Verschmutzungsgrades oder Hygienezustandes an einem Indikatorbauteil, kann zudem einen Schritt umfassen, wobei aus Abweichungen in der Reinigbarkeit oder Verschmutzbarkeit des Indikatorbauteils im Vergleich zur Reinigbarkeit oder Verschmutzbarkeit der schwer zu reinigenden Stelle der Prozessanlage, z.B. Lebensmittelanlage, ein Verhältnisfaktor ermittelt wird, welcher bei der Durchführung eines Reinigungsprozesses berücksichtigt wird.

Zur Bestimmung bzw. Messung des Verschmutzungsgrades oder Hygienezustandes können Belagssensoren der oben beschriebenen Art eingesetzt werden, also beispielsweise kapazitive Belagssensor, oder Ultraschall-Belagssensoren, oder optische Belagssensoren, oder thermische Belagssensoren, oder Belagssensoren mit Konduktionsmessung, oder Belagssensoren mit Fluoreszenzmessung, oder Belagssensoren mit Absorptionsmessung.

Besagte beispielhafte Belagssensoren können in die zu untersuchende Stelle, bzw. in die zu messende Oberfläche, integriert werden.

Alternativ oder zusätzlich können z.B. optische Messverfahren mit unterschiedlichen Wellenlängenbereichen eingesetzt werden. Hierzu kann z.B. mindestens ein Schauglas, bzw. eine optische Öffnung, so angebracht werden, dass mit einer Auswerteeinheit (z.B. einer optischen Einheit, z.B. optischer Belagssensor) die zu untersuchende Stelle oder das Indikatorbauteil beobachtet werden kann. Wellenlängenbereiche für derartige optische Kontrollen bzw. Messverfahren können dabei z.B. im UVA/UVB-Bereich zwischen 300 und 400 nm, und/oder im sichtbaren Bereich zwischen 400 und 700 nm, und/oder im NIR-Bereich zwischen 700 und 1000 nm und/oder im SWIR-Bereich zwischen 1000 und 2500 nm liegen.

Während der Reinigung ist es dann beispielsweise möglich, den Reinheitsgrad der zu untersuchenden Stelle bzw. des Indikatorbauteils mit Hilfe der optischen Einheit zu überwachen und man kann so lange reinigen, bis dieses komplett sauber ist. Die Optische Auswertung kann dabei nach dem Prinzip der direkten Beobachtung (z.B. per Videokamera), der Reflexion (z.B. mittels Floureszensmessung) oder im Durchlichtverfahren durchgeführt werden.

Da wie erwähnt, das optionale Indikatorbauteil der am schwersten zu reinigenden Bauteil und/oder der Stelle mit verstärkter Belagsbildung entsprechen kann, ist davon auszugehen, dass die gesamte Anlage den gewünschten Zustand hat, wenn dieses Bauteil sauber ist.

Die hier beschriebenen Mittel und Verfahrensschritte sind alle miteinander kombinierbar und können synergetisch dahingehend zusammenwirken, das Überwachen des Hygienezustandes einer Prozessanlage, z.B. Lebensmittelanlage, und das Überwachen und Steuern eines Reinigungsprozesses der Prozessanlage, z.B. Lebensmittelanlage, zu verbessern. Insbesondere kann beispielweise eine Reinigung der Anlage nach Bedarf, bzw. angepasst an den aktuellen Verschmutzungsgrad der Anlage, erfolgen. So kann unter anderem eine sichere Produktion bei möglichst niedrigem Reinigungsmedieneinsatz gewährleistet werden. Besondere Bedeutung hat die Erfindung bzw. haben die beschrieben Mittel beispielsweise auch auf eine eventuelle Verkürzung der Reinigungszeit, wodurch die Produktionszeit der Anlage maximiert werden kann.

Folgende Figuren stellen beispielhaft dar:
- **Fig.1a:**: Beispielhafte Messbereiche zweier beispielhafter Belagssensoren
- **Fig.1b:**: Weiteres Beispiel für Messbereiche zweier beispielhafter Belagssensoren
- **Fig. 2:**: Beispielhafte Anordnung eines beispielhaften thermischen Belagssensors
- **Fig. 3:**: Beispielhaftes beaufschlagtes Temperaturprofil und beispielhaft ausgelöstes gemessenes zeitlich veränderliches Temperaturprofil
- **Fig. 4:**: Beispielhafte Anordnung eines beispielhaften optischen Belagssensors

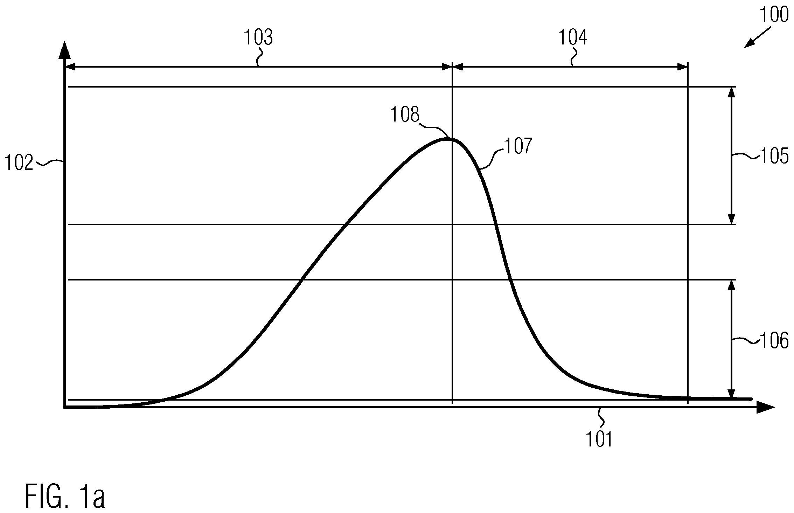

Die **Fig. 1a** stellt beispielhaft eine mögliche Messbereichsaufteilung 100 beispielhafter Messbereiche 105,106 zweier beispielhafter Belagssensoren dar, wobei die Belagssensoren (nicht dargestellt) nach einer der oben beschriebenen Arten aufgebaut sein können, bzw. nach den verschiedenen oben beschriebenen Messprinzipien arbeiten können.

Zudem ist ein beispielhafter Verlauf 107 der Verschmutzung eines zu untersuchenden Teils einer Prozessanlage, z.B. Lebensmittelanlage, dargestellt. Dieser Verlauf 107 kann beispielsweise den Verlauf bzw. die Änderung der Schichtdicke 102 eines Belags in Abhängigkeit der Zeit 101 darstellen.

Dabei sind beispielsweise zwei verschiedene Prozess- bzw. Zeitabschnitte 103, 104 dargestellt, wobei der erste Prozess- bzw. Zeitabschnitt 103 beispielsweise die Belagsschichtdickenänderung bei Belagsbildung in einer Prozessanlage, z.B. Lebensmittelanlage, während der Produktion darstellen kann, und der zweite Prozess- bzw. Zeitabschnitt 104 die Belagsschichtdickenänderung bei Belagsabbau bzw. Belagsentfernung während einer Reinigung darstellen kann.

Dabei können, wie dargestellt, beispielsweise die nach beispielsweise unterschiedlichen Messprinzipien arbeitenden Belagssensoren Messbereiche 105, 106 aufweisen, die sich nicht überlappen.

Dabei kann ein beispielhafter erster Messbereich 105 eines ersten Belagssensors Belagsschichtdicken mit einer maximal tolerierbaren Belagsschichtdicke 108 umfassen.

So kann z.B. bei Feststellung dass die Belagsschichtdicke die vorgegebene maximal tolerierbare Belagsschichtdicke 108 überschreitet eine Reinigung 104 eingeleitet werden.

Der dargestellte beispielhafte zweite Messbereich 106 eines zweiten Belagssensors kann beispielsweise Belagschichtdicken von nahe oder gleich Null umfassen.

Beide Messbereiche 105, 106 können zudem weiter in verschiedenen Unterbereiche unterteilt sein (nicht dargestellt). Beispielsweise kann Messbereich 105 einen oberen und unteren Messbereich enthalten. Ebenso kann Messbereich 106 einen oberen und unteren Messbereich enthalten.

So kann z.B. der Verlauf der Reinigung bzw. der Belagsentfernung überwacht werden und bei Feststellung einer zufriedenstellenden Entfernung bzw. hinreichenden Verminderung des Belags die Reinigung 104 beendet werden und die Prozessanlage, z.B. Lebensmittelanlage, wieder in Produktionsbetrieb 103 gehen.

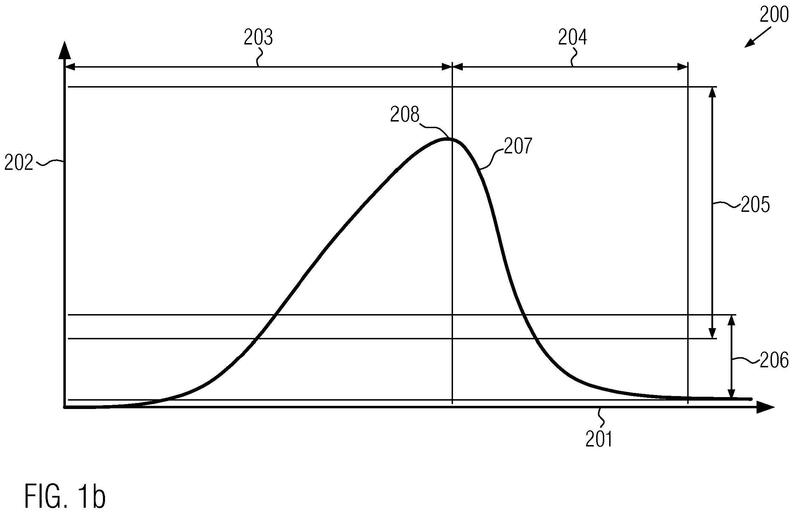

Die **Fig. 1b** stellt beispielhaft eine weitere mögliche Messbereichsaufteilung 200 beispielhafter Messbereiche 205, 206 zweier beispielhafter Belagssensoren dar, wobei die Belagssensoren (nicht dargestellt) wieder nach einer der oben beschriebenen Arten aufgebaut sein können, bzw. nach den verschiedenen oben beschriebenen Messprinzipien arbeiten können.

Dabei sind analog zu Fig.1a beispielsweise zwei verschiedene Prozess- bzw. Zeitabschnitte 203, 204 dargestellt, wobei der erste Prozess- bzw. Zeitabschnitt 203 beispielsweise die Belagsschichtdickenänderung bei Belagsbildung in einer Prozessanlage, z.B. Lebensmittelanlage, während der Produktion darstellen kann, und der zweite Prozess- bzw. Zeitabschnitt 204 die Belagsschichtdickenänderung bei Belagsabbau während einer Reinigung darstellen kann.

Dabei können, wie dargestellt, beispielsweise die nach beispielsweise unterschiedlichen Messprinzipien arbeitenden Belagssensoren im Unterschied zu Fig. 1a jedoch Messbereiche 205, 206 aufweisen, die sich überlappen.

Dabei kann ein beispielhafter erster Messbereich 205 eines ersten Belagssensors wiederum Belagsschichtdicken mit einer maximal tolerierbaren Belagsschichtdicke 208 umfassen.

So kann z.B. bei Feststellung, dass die Belagsschichtdicke die vorgegebene maximal tolerierbare Belagsschichtdicke 208 überschreitet, eine Reinigung 204 eingeleitet werden.

Der dargestellte beispielhafte zweite Messbereich 206 eines zweiten Belagssensors kann beispielsweise Belagschichtdicken von nahe oder gleich Null umfassen.

Beide Messbereiche 205, 206 können zudem analog zu Fig. 1a weiter in verschiedenen Unterbereiche unterteilt sein (nicht dargestellt). Beispielsweise kann Messbereich 205 einen oberen und unteren Messbereich enthalten. Ebenso kann Messbereich 206 einen oberen und unteren Messbereich enthalten.

Die beispielhafte mögliche Überlappung der Messbereiche 205, 206 kann dabei unter anderem eine genauere bzw. lückenlosere Überwachung des Hygienezustandes einer Prozessanlage, z.B. Lebensmittelanlage, ermöglichen.

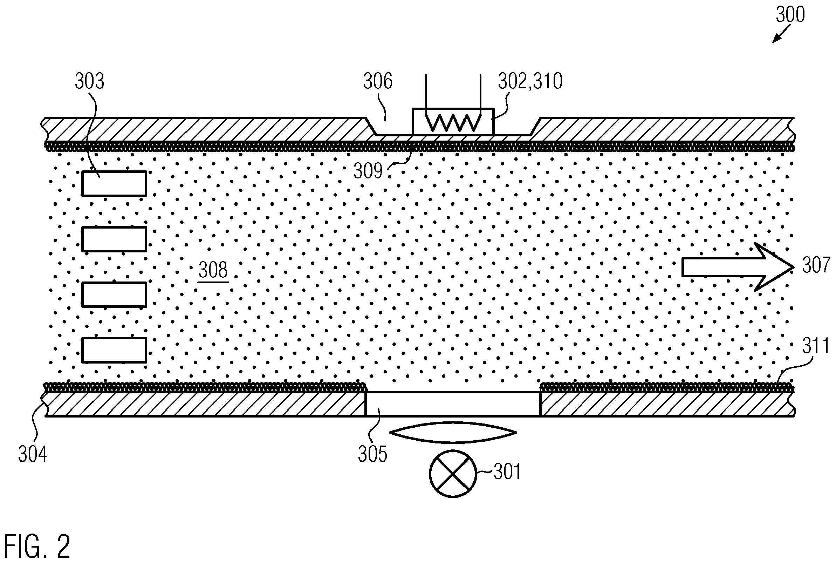

Die **Fig. 2** stellt beispielhaft eine mögliche Anordnung eines beispielhaften thermischen Belagssensors 310 für ein System zum Überwachen des Hygienezustandes einer Prozessanlage, z.B. Lebensmittelanlage, und zum Überwachen und Steuern eines Reinigungsprozesses einer Prozessanlage, z.B. Lebensmittelanlage, dar, der beispielsweise einen Temperatursensor 302 und wenigstens einen Temperaturemitter 301 aufweisen kann, konfiguriert zur Einkopplung von Wärmeenergie, z.B. zur Beaufschlagung eines zeitlich veränderlichen, z.B. periodisch veränderlichen, Temperaturprofiles, in eine zu untersuchende Stelle 306 eines Teils 304, z.B. einer Rohrwandung, der Prozessanlage, z.B. Lebensmittelanlage.

Anstelle eines Wärmestrahlers ist es auch denkbar, einen Ultraschall-Transducer zur Einkopplung von Wärmeenergie bzw. zur Beaufschlagung eines zeitlich veränderlichen Temperaturprofiles einzusetzen.

Die Temperaturverlaufsmessung der durch die Beaufschlagung eines zeitlich veränderlichen Temperaturprofiles ausgelösten Temperaturänderung, kann dabei an einer speziell ausgestaltete zu untersuchende Stelle bzw. Messstelle 306 erfolgen.

Hierzu ist beispielsweise denkbar, dass, wie dargestellt, eine Außenwandung der Messstelle verjüngt ist. Dies kann die Messung der ausgelösten Temperaturänderung vereinfachen, ohne eine Beeinträchtigung der Homogenität der Messstelle, insbesondere ohne Beeinträchtigung der Homogenität einer Innenwandung der Messstelle, zu verursachen, so dass die Messstelle das gleiche Anlagerungsverhalten für Beläge 310 wie an den umgebenden normalen Bauteilen aufweisen kann.

Wie dargestellt kann beispielsweise der Temperaturemitter 301 gegenüber dem Temperatursensor 302 des beispielhaften thermischen Belagssensors 310 angeordnet sein.

Alternativ und/oder zusätzlich, kann die Beaufschlagung eines zeitlich veränderlichen Temperaturprofiles in eine zu untersuchende Stelle 306 der Prozessanlage, z.B. Lebensmittelanlage, durch periodisches Heizen und Kühlen eines Mediums 308, z.B. Reinigungsmediums, durch optionale Heiz- bzw. Kühlelemente 303 erfolgen.

Besagte optionale Heiz- bzw. Kühlelemente 303 können dabei vor der zu untersuchende Stelle angeordnet sein, so dass beispielsweise das Medium 308 vor Erreichen der untersuchende Stellen mit einem zeitlich veränderlichen Temperaturprofil beaufschlagt werden kann.

Zudem kann beispielsweise die Beaufschlagung des zeitlich veränderlichen Temperaturprofils bei konstantem Medienfluss 307 erfolgen.

Bei Einsatz der optionalen Heiz- bzw. Kühlelemente 303 kann unter Umständen der thermische Belagssensor 310 auch ohne einen dem Temperatursensor 302 gegenüberliegenden (zusätzlichen)Temperaturemitter betrieben werden.

Die Beaufschlagung eines zeitlich veränderlichen Temperaturprofiles in eine zu untersuchende Stelle 306 der Prozessanlage, z.B. Lebensmittelanlage, durch Wärmebestrahlung, kann von einer der zu untersuchenden Stelle gegenüberliegenden Stelle 305, der Einstrahlstelle, durch ein Medium 308, z.B. Produkt- oder Reinigungsmedium, hindurch erfolgen, wobei die die Einstrahlstelle beispielsweise so ausgelegt sein kann, dass dort eine verminderte Belagsbildung erfolgt bzw. vorhanden ist, wobei dies ggf. durch eine Kühlung der Einstrahlstelle, z.B. Peltierkühlung (nicht dargestellt), erreicht werden kann.

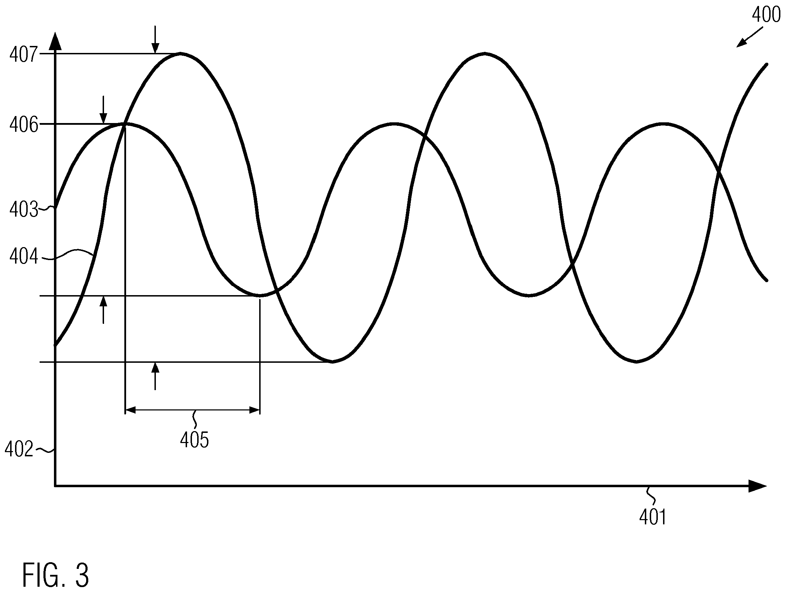

Die **Fig. 3** stellt beispielhafte Wärmeprofile bzw. Temperaturprofile 400 dar, also die Abhängigkeit einer Wärme bzw. Temperatur 402 von der Zeit 401.

Dabei stellt das Profil 403 beispielsweise ein, z.B. durch oben beschrieben Mittel beaufschlagtes zeitlich veränderliches Temperaturprofil 403 dar, welches auf eine zu untersuchende Stelle einer (nicht dargestellten) Prozessanlage, z.B. Lebensmittelanlage, beaufschlagt werden kann.

Profil 403 kann auch als beispielhaftes Anregungsprofil bezeichnet werden.

Wie dargestellt, ist das beispielhafte veränderliche Temperaturprofil 403 bzw. Anregungsprofil periodisch veränderlich und weist eine beispielhafte Amplitude 406 auf.

Das Profil 404 stellt dabei beispielsweise, die z.B. von einem thermischen Belagssensor gemessene und durch das Anregungsprofil 403 ausgelöste Temperaturänderung, bzw. das z.B. von einem thermischen Belagssensor gemessene und durch das Anregungsprofil 403 ausgelöste Temperaturprofil, an dem zu untersuchendem Teil / der zu untersuchenden Stelle der Prozessanlage, z.B. Lebensmittelanlage, dar.

Profil 404 kann auch als beispielhaftes Messprofil bezeichnet werden.

Wie oben beschrieben, kann aus dem beispielhaften Messprofil 404 durch die Messung des Betrags bzw. der Amplitude 407 und der Verzögerung bzw. des Phasenunterschieds 405 des Messprofils 404 im Vergleich zum Anregungsprofil, auf das Vorhandensein und die Stärke der Belagsbildung bzw. auf die Stärke der Belagsschichtdicke geschlossen werden.

Dabei kann beispielsweise der Zusammenhang zwischen gemessener Amplitude 407 und gemessenen Phasenunterschied 405 dabei eine Funktion der Temperaturleitfähigkeit des Belags und/oder der Belagsdicke und/oder dem Messprofil 404 sein. So kann beispielsweise bei einer vergleichsweise niedrigeren Temperaturleitfähigkeit mit einer Verminderung der Amplitude und größeren Phasenverschiebung bzw. größerem Phasenunterschied 405 zu rechnen sein. Ebenso kann eine größere Belagsdicke die Amplitude 407 vermindern und die Phasenverschiebung bzw. den Phasenunterschied 405 vergrößern.

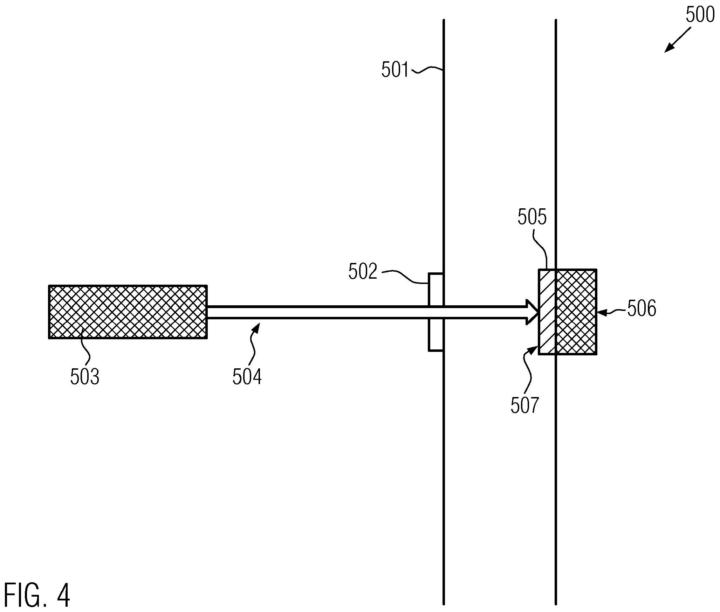

Die **Fig. 4** stellt beispielhaft eine mögliche Anordnung 500 eines beispielhaften optischen Belagssensors zusammen mit einem beispielhaften Indikatorbauteil 505 dar.

Das Indikatorbauteil 505 kann dabei beispielsweise an einer schwer zu reinigenden Stelle der Prozessanlage, z.B. Lebensmittelanlage, angebracht sein.

Alternativ kann das Indikatorbauteil 505 an einer beliebigen zu untersuchenden Stelle 507 der zu untersuchenden Prozessanlage, z.B. Lebensmittelanlage, angebracht sein, beispielsweise an einer Stelle einer Rohrwandung 501, und wobei das Indikatorbauteil 505 dazu konfiguriert sein kann, eine schwer zu reinigende Stelle der Prozessanlage, z.B. Lebensmittelanlage, und/oder eine Stelle mit verstärkter Belagsbildung zu simulieren.

Zur Verbesserung der Simulation einer schwer zu reinigenden Stelle, bzw. einer Stelle mit verstärkter Belagsbildung, kann das Indikatorbauteil 505 optional auch über ein Heizelement beheizt werden.

Auch ist denkbar, dass beispielsweise das Indikatorbauteil 505 beheizbar sein kann mit einem zeitlich veränderlichen Temperaturprofil.

Zu Messung des Verschmutzungsgrades oder Hygienezustandes, bzw. zur Messung der Belagsbildung an der zu untersuchenden Stelle der Prozessanlage, z.B. Lebensmittelanlage, kann das Indikatorbauteil 505 mit optischen Messmethoden beobachtet werden.

Hierzu kann beispielsweise ein optischer Belagssensor 503, z.B. eine Kamera, welcher gegenüber dem Indikatorbauteil 505 angeordnet werden kann, über einen Messlichtstrahl 504 die Belagsbildung auf dem Indikatorbauteil optisch erfassen und messen.

Der Messlichtstrahl 503 kann dabei z.B. über eine optische Öffnung, z.B. ein Schauglas, angebracht in/an einem dem Indikatorbauteil 505 gegenüberliegenden Teil der zu untersuchenden Anlagenstelle, das Indikatorbauteil erfassen.

Es folgen 5 Blatt mit Figuren Fig. 1a, Fig. 1b, Fig. 2, Fig. 3 und Fig. 4.

Die Bezugszeichen sind dabei wie folgt belegt.
- **100**: Beispielhafte Messbereichsaufteilung
- **101**: Beispielhafte Abszissenachse, z.B. Zeitachse
- **102**: Beispielhafte Ordinatenachse, z.B. Achse die den Verschmutzungsgrad bzw. Reinigungsgrad misst, z.B. die Belagsschichtdicke und/oder andere physikalische Eigenschaften eines Belags oder Produktes
- **103**: Beispielhafter (erster) Anlageprozessabschnitt, z.B. Lebensmittelanlageprozessabschnitt, z.B. Produktionsabschnitt
- **104**: Beispielhafter (zweiter) Anlageprozessabschnitt, z.B. Lebensmittelanlageprozessabschnitt, z.B. Reinigungsabschnitt
- **105**: Beispielhafter (erster) Messbereich eines (ersten) Belagssensors
- **106**: Beispielhafter (zweiter) Messbereich eines (zweiten) Belagssensors
- **107**: Beispielhafter Verlauf der Verschmutzung eines zu untersuchenden Teils einer Prozessanlage, z.B. Lebensmittelanlage, beispielhafter Verlauf der Belagsbildung bzw. Belagsentfernung, z.B. Verlauf der Belagsschichtdicke
- **108**: Beispielhafte maximal tolerierbare Belagsschichtdicke
- **200**: Beispielhafte Messbereichsaufteilung
- **201**: Beispielhafte Abszissenachse, z.B. Zeitachse
- **202**: Beispielhafte Ordinatenachse, z.B. Achse die den Verschmutzungsgrad bzw. Reinigungsgrad misst, z.B. die Belagsschichtdicke und/oder andere physikalische Eigenschaften eines Belags oder Produktes
- **203**: Beispielhafter (erster) Anlageprozessabschnitt, z.B. Lebensmittelanlageprozessabschnitt, z.B. Produktionsabschnitt
- **204**: Beispielhafter (zweiter) Anlageprozessabschnitt, z.B. Lebensmittelanlageprozessabschnitt, z.B. Reinigungsabschnitt
- **205**: Beispielhafter (erster) Messbereich eines (ersten) Belagssensors
- **206**: Beispielhafter (zweiter) Messbereich eines (zweiten) Belagssensors
- **207**: Beispielhafter Verlauf der Verschmutzung eines zu untersuchenden Teils einer Prozessanlage, z.B. Lebensmittelanlage, beispielhafter Verlauf der Belagsbildung bzw. Belagsentfernung, z.B. Verlauf der Belagsschichtdicke
- **208**: Beispielhafte maximal tolerierbare Belagsschichtdicke
- **300**: Beispielhafte Anordnung eines beispielhaften thermischen Belagssensors
- **301**: Beispielhafter Teil eines beispielhaften thermischen Belagssensors, z.B. Temperaturemitter, z.B. Wärmestrahler und/oder Ultraschall-Transducer
- **302**: Beispielhafter Teil eines beispielhaften thermischen Belagssensors, z.B. Temperatursensor
- **303**: Beispielhafte Heiz- und/oder Kühlelemente, z.B. auch als Teil des Temperaturemitters auffassbar
- **304**: Beispielhafter Teil der zu untersuchenden Lebensmittelanlage, z.B. Rohrwandung
- **305**: Beispielhafte Stelle zur Einkopplung / Einstrahlung / Beaufschlagung von Wärmeenergie
- **306**: Beispielhafte Messstelle / beispielhafte zu untersuchende Stelle
- **307**: Beispielhafter Medienfluss
- **308**: Beispielhaftes Medium, z.B. Produkt und/oder Reinigungsmedium
- **309**: Verschmutzung, z.B. Belag
- **310**: Beispielhafter thermischer Belagssensor
- **311**: Verschmutzung, z.B. Belag
- **400**: Beispielhafte Temperaturprofile
- **401**: Beispielhafte Abszissenachse, z.B. Zeitachse
- **402**: Beispielhafte Ordinatenachse, z.B. Temperaturachse bzw. Wärmeenergieachse
- **403**: Beispielhaftes beaufschlagtes zeitlich veränderliches Temperaturprofil, beispielhaftes Anregungsprofil
- **404**: Beispielhaftes gemessenes, durch Anregung, z.B. Anregungsprofil 403, ausgelöstes gemessenes Temperaturprofil an zu untersuchendem Teil / zu untersuchender Stelle der Anlage, beispielhaftes Messprofil
- **405**: Beispielhafte Verzögerung, Beispielhafter Phasenunterschied zwischen Anregungsprofil und Messprofil
- **406**: Beispielhafte Amplitude des beaufschlagten zeitlich veränderlichen Temperaturprofiles
- **407**: Beispielhafte Amplitude des gemessenen durch Anregung, z.B. Anregungsprofil 403, ausgelösten gemessenen Temperaturprofils
- **500**: Beispielhafte Anordnung eines beispielhaften optischen Belagssensors
- **501**: Beispielhaftes zu untersuchendes Teil, z.B. Rohrwandung, einer Prozessanlage, z.B. Lebensmittelanlage
- **502**: Beispielhafte Stelle zur Einkopplung / Einstrahlung eines Messstrahls, z.B. eines Lichtstrahles, beispielhafte optische Öffnung bzw. Schauglas
- **503**: Beispielhafter Belagssensor, z.B. optischer Belagssensor
- **504**: Beispielhafter Messstrahl, z.B. Lichtstrahl
- **505**: Beispielhaftes Indikatorbauteil bzw. Referenzbauteil
- **506**: Beispielhaftes optionales Heizelement
- **507**: Beispielhafte zu untersuchende Stelle, z.B. schwer zu reinigende Stelle:

## Patentansprüche

1. System zum Überwachen des Hygienezustandes einer Prozessanlage und zum Überwachen und Steuern eines Reinigungsprozesses der Prozessanlage umfassend:
wenigstens zwei Belagssensoren für Messungen der Belagsschichtdicke auf Teilen der Prozessanlage (304), wobei die wenigstens zwei Belagssensoren verschiedene Messbereiche (105, 106) aufweisen.

2. System nach Anspruch 1, wobei wenigstens ein Belagssensor ein kapazitiver Belagssensor ist und wenigstens ein Belagssensor ein Ultraschall-Belagssensor ist, und/oder
wobei alternativ oder zusätzlich wenigstens ein Belagssensor ein optischer Belagssensor oder ein thermischer Belagssensor oder ein Belagssensor mit Konduktionsmessung oder ein Belagssensor mit Fluoreszenzmessung oder ein Belagssensor mit Absorptionsmessung ist.

3. System nach einem der vorherigen Ansprüche, wobei sich die Messbereiche (205, 206) der Belagssensoren teilweise überlappen.

4. System nach einem der vorherigen Ansprüche, wobei der Messbereich (106) wenigstens eines Belagssensors Belagschichtdicken von nahe oder gleich Null umfasst und/oder der Messbereich (105) wenigstens eines Belagssensors Belagschichtdicken mit einer maximal tolerierbaren Belagsschichtdicke umfasst.

5. System nach einem der vorherigen Ansprüche, wobei wenigstens ein Belagssensor alternativ oder zusätzlich konfiguriert ist zur Messung einer physikalischen Eigenschaft eines die zu untersuchende Stelle durchströmenden Mediums (308), beispielsweise konfiguriert ist zur Messung der elektrischen Leitfähigkeit eines die zu untersuchende Stelle durchströmenden Mediums (308).

6. System nach einem der vorherigen Ansprüche, wobei wenigstens ein Belagssensor an einer schwer zu reinigenden Stelle, z.B. Hochtemperaturbereiche von Wärmetauschern, Vordenaturierungsstufen, Ringkessel oder Zentralkessel einer Befüllungsvorrichtung, Füllventile, Puffertanks, Totstellen, Rohrerweiterungen, Hinterschneidungen, Übergänge von Dichtungen zu Materialen, und/oder wobei wenigstens ein Belagssensor an einer Stelle mit im Vergleich zu anderen Stellen der Prozessanlage, z.B. Lebensmittelanlage, verstärkter Belagsbildung angebracht ist.

7. System nach einem der vorherigen Ansprüche, wobei wenigstens ein Belagssensor beheizbar ist.

8. System nach einem der vorherigen Ansprüche, wobei wobei die Prozessanlage eine Lebensmittelanlage ist.

9. Verfahren zum Überwachen des Hygienezustandes einer Prozessanlage und zum Überwachen und Steuern eines Reinigungsprozesses der Prozessanlage umfassend:
Überwachung des Hygienezustandes bei Produktion durch Überwachen der Belagsbildung an wenigstens einem Teil der Prozessanlage während der Produktion, mittels wenigstens eines Belagssensors für Messungen der Belagsschichtdicke, dessen Messbereich (105) Belagsschichtdicken mit einer vorgegebenen Belagsschichtdicke (108) umfasst,
Einleitung eines Reinigungsprozesses der Prozessanlage bei Feststellung durch den wenigstens einen Belagssensor, dass die Belagsschichtdicke die vorgegebene Belagsschichtdicke (108) überschreitet,
Überwachung und/oder Beendigung des eingeleiteten Reinigungsprozesses durch wenigstens einen weiteren Belagssensor für Messungen der Belagsschichtdicke, dessen Messbereich verschieden von dem Messbereich des ersten Belagssensor ist.

10. Verfahren nach dem vorherigen Anspruch, wobei der Belagssensor für Messungen der Belagsschichtdicke, dessen Messbereich (105) Belagsschichtdicken mit einer vorgegebenen Belagsschichtdicke (108) umfasst, einen Messbereich mit einer vorgegebenen maximal tolerierbaren, Belagsschichtdicke (108) umfasst und/oder
wobei der weitere Belagssensor einen Messbereich aufweist, der Belagschichtdicken von nahe oder gleich Null umfasst.

11. Verfahren nach dem vorherigen Anspruch, wobei das Überwachen der Belagsbildung an wenigstens einem Teil der Prozessanlage während der Produktion zunächst durch den Belagssensor erfolgt, dessen unterer Messbereich Belagschichtdicken von nahe oder gleich Null umfasst, und wobei erst bei Erreichen des oberen Endes des Messbereiches des Belagssensors mit unterem Messbereich von Belagschichtdicken von nahe oder gleich Null, die Überwachung des Hygienezustandes bei Produktion umgeschaltet wird, auf den Belagssensors, dessen Messbereich (105) Belagschichtdicken mit einer vorgegebenen maximal tolerierbaren Belagsschichtdicke umfasst.

12. Verfahren nach einem der vorherigen Verfahrensansprüche, wobei die Überwachung des eingeleiteten Reinigungsprozesses zunächst durch den Belagssensor erfolgt, dessen unterer Messbereich Belagschichtdicken mit einer vorgegebenen maximal tolerierbaren Belagsschichtdicke umfasst, und wobei erst bei Erreichen des unteren Endes des Messbereiches des Belagssensor mit Messbereich (105) für Belagschichtdicken mit einer vorgegebenen maximal tolerierbaren Belagsschichtdicke (108), die Überwachung des eingeleiteten Reinigungsprozesses umgeschaltet wird, auf den Belagssensor dessen unterer Messbereich Belagschichtdicken von nahe oder gleich Null umfasst.

13. Verfahren nach einem der vorherigen Verfahrensansprüche, wobei die Überwachung des eingeleiteten Reinigungsprozesses zusätzlich die Überwachung eines Ausspülens, z.B. mit Wasser, eines Reinigungsmediums umfasst, wobei die Überwachung des Ausspülens mittels eines Sensors erfolgt, der dazu konfiguriert ist, eine physikalischen Eigenschaft eines die zu reinigende Stelle durchströmenden Mediums (308) zu messen, beispielsweise konfiguriert dazu ist die elektrische Leitfähigkeit eines die zu reinigende Stelle durchströmenden Mediums (308) zu messen.

14. Verfahren nach einem der vorherigen Verfahrensansprüche, wobei die Prozessanlage eine Lebensmittelanlage ist.

## Claims

1. A system for monitoring the hygienic status of a process plant and for monitoring and controlling a cleaning process of the process plant comprising:
at least two film sensors for measuring the thickness of films on parts of the process plant (304) wherein the at least two film sensors have different measuring regions (105, 106).

2. The system according to claim 1, wherein at least one film sensor is a capacitive film sensor and at least one film sensor is an ultrasonic film sensor and/or
wherein alternatively or additionally at least one film sensor is an optical sensor or a thermal film sensor or a film sensor using conductivity measurement or a film sensor using fluorescence measurement or a film sensor using absorption measurement.

3. The system according to any one of the preceding claims, wherein the measuring regions (205, 206) of the film sensors partially overlap.

4. The system according to any one of the preceding claims, wherein the measuring region (106) of at least one film sensor comprises film thicknesses near or equal to 0 and/or the measuring region (105) of at least one film sensor comprises film thicknesses including a maximum tolerable film thickness.

5. The system according to any one of the preceding claims, wherein at least one film sensor is alternatively or additionally configured to measure a physical property of a medium (308) flowing through the position to be inspected, and is, for example, configured to measure the electric conductivity of a medium (308) flowing through the position to be inspected.

6. The system according to any one of the preceding claims, wherein at least one film sensor is mounted at a hard-to-be cleaned position such as high-temperature regions of heat exchangers, pre-denaturation stages, ring bowls or central bowls of a filling apparatus, filling valves, buffering tanks, wake spaces, pipe expansions, undercuts, interfaces between sealings and materials, and/or wherein at least one film sensor is mounted at a position having stronger film formation than other positions of the process plant, for instance a food processing plant.

7. The system according to any one of the preceding claims, wherein at least one film sensor can be heated.

8. The system according to any one of the preceding claims, wherein the process plant is a food processing plant.

9. A method for monitoring the hygienic status of a process plant and for monitoring and controlling a cleaning process of the process plant comprising:
monitoring the hygienic status during production by monitoring the formation of films on at least a part of the process plant during production by means of at least one film sensor for measuring the film thickness the measuring region (105) of which comprises film thicknesses including a predetermined film thickness (108), initiating a cleaning process of the process plant when the at least one film sensor determines that the film thickness exceeds the predetermined film thickness (108), monitoring and/or stopping the initiated cleaning process by at least one further film sensor for measuring the film thickness the measuring region of which differs from the measuring region of the first film sensor.

10. The method according to the preceding claim, wherein the film sensor for measuring the film thickness the measurement region (105) of which comprises film thicknesses including a predetermined film thickness (108) comprises a measuring region including a predetermined maximum tolerable film thickness (108) and/or
wherein the further film sensor has a measuring region near or equal to 0.

11. The method according to the preceding claim, wherein the monitoring the film formation on at least a part of the process plant during production is first carried out by the film sensor the lower measuring region of which comprises film thicknesses near or equal to 0, and wherein only after reaching the upper end of the measuring region of the film sensor having the lower measuring region of film thicknesses near or equal to 0 monitoring the hygienic status during production is switched to the film sensor the measuring region (105) of which comprises film thicknesses including a predetermined maximum tolerable film thickness.

12. The method according to any one of the preceding method claims, wherein the monitoring the initiated cleaning process is first carried out by the film sensor the lower measuring region of which comprises film thicknesses including a predetermined maximum tolerable film thickness, and wherein only after reaching the lower end of the measuring region of the film sensor having the measuring region (105) for film thicknesses including a predetermined maximum tolerable film thickness (108) the monitoring of the initiated cleaning process is switched to the film sensor the lower measuring region of which comprises film thicknesses near or equal to 0.

13. The method according to any one of the preceding method claims, wherein the monitoring of the initiated cleaning process further comprises monitoring a purging of a cleaning medium for instance with water, wherein monitoring the purging is carried out by means of a sensor configured to measure a physical property of the medium (308) flowing through the position to be cleaned, for instance being configured to measure the electric conductivity of a medium (308) flowing through the position to be cleaned.

14. The method according to any one of the preceding claims, wherein the process plant is a food processing plant.

## Revendications

1. Système pour surveiller l'état d'hygiène d'une installation de traitement et pour surveiller et commander un processus de nettoyage de l'installation de traitement, comprenant :
au moins deux capteurs de dépôt pour mesurer l'épaisseur de la couche de dépôt sur des parties de l'installation de traitement (304), les au moins deux capteurs de dépôt présentant différentes plages de mesure (105, 106).

2. Système selon la revendication 1, dans lequel au moins un capteur de dépôt est un capteur de dépôt capacitif et au moins un capteur de dépôt est un capteur de dépôt à ultrasons, et/ou
dans lequel, en variante ou en plus, au moins un capteur de dépôt est un capteur de dépôt optique ou un capteur de dépôt thermique ou un capteur de dépôt avec mesure de conductivité ou un capteur de dépôt avec mesure de fluorescence ou un capteur de dépôt avec mesure d'absorption.

3. Système selon l'une des revendications précédentes, dans lequel les plages de mesure (205, 206) des capteurs de dépôt se chevauchent partiellement.

4. Système selon l'une des revendications précédentes, dans lequel la plage de mesure (106) d'au moins un capteur de dépôt comprend des épaisseurs de couche de dépôt proches de zéro ou égales à zéro et/ou la plage de mesure (105) d'au moins un capteur de dépôt comprend des épaisseurs de couche de dépôt avec une épaisseur de couche de dépôt maximale tolérable.

5. Système selon l'une quelconque des revendications précédentes, dans lequel au moins un capteur de dépôt est configuré, en variante ou en plus, pour mesurer une propriété physique d'un milieu (308) traversant l'endroit à examiner, par exemple configuré pour mesurer la conductivité électrique d'un milieu (308) traversant l'endroit à examiner.

6. Système selon l'une des revendications précédentes, dans lequel au moins un capteur de dépôt est placé à un endroit difficile à nettoyer, par exemple des zones à haute température d'échangeurs de chaleur, des étapes de pré-dénaturation, des cuves annulaires ou des cuves centrales d'un dispositif de remplissage, des vannes de remplissage, des cuves tampons, des points morts, des élargissements de tubes, des contre-dépouilles, des transitions entre des joints et des matériaux, et/ou dans lequel au moins un capteur de dépôt est placé à un endroit où la formation de dépôt est plus importante par rapport à d'autres endroits de l'installation de traitement, par exemple une installation alimentaire.

7. Système selon l'une quelconque des revendications précédentes, dans lequel au moins un capteur de dépôt peut être chauffé.

8. Système selon l'une quelconque des revendications précédentes, dans lequel l'installation de traitement est une installation alimentaire.

9. Procédé de surveillance de l'état d'hygiène d'une installation de traitement et de surveillance et de commande d'un processus de nettoyage de l'installation de traitement comprenant :
la surveillance de l'état d'hygiène lors de la production, par la surveillance de la formation d'un dépôt sur au moins une partie de l'installation de traitement pendant la production, au moyen d'au moins un capteur de dépôt pour des mesures de l'épaisseur de couche de dépôt, dont la plage de mesure (105) comprend des épaisseurs de couche de dépôt ayant une épaisseur de couche de dépôt (108) prédéterminée,
le lancement d'un processus de nettoyage de l'installation de traitement lors de la constatation par le au moins un capteur de dépôt que l'épaisseur de la couche de dépôt excède l'épaisseur de couche de dépôt (108) prédéterminée,
la surveillance et/ou l'achèvement du processus de nettoyage initié par au moins un autre capteur de dépôt pour des mesures de l'épaisseur de la couche de dépôt, dont la plage de mesure est différente de la plage de mesure du premier capteur de dépôt.

10. Procédé selon la revendication précédente, dans lequel le capteur de dépôt pour des mesures de l'épaisseur de la couche de dépôt, dont la plage de mesure (105) comprend des épaisseurs de couche de dépôt avec une épaisseur de couche de dépôt (108) prédéfinie, comprend une plage de mesure avec une épaisseur de couche de dépôt (108) maximale tolérable prédéfinie et/ou
dans lequel l'autre capteur de dépôt présente une plage de mesure comprenant des épaisseurs de couche de dépôt proches de, ou égales à, zéro.

11. Procédé selon la revendication précédente, dans lequel la surveillance de la formation de dépôt sur au moins une partie de l'installation de traitement pendant la production est tout d'abord effectuée par le capteur de dépôt dont la plage de mesure inférieure comprend des épaisseurs de couche de dépôt proches ou égales à zéro, et où ce n'est que lorsque l'extrémité supérieure de la plage de mesure du capteur de dépôt avec une plage de mesure inférieure d'épaisseurs de couche de dépôt proches ou égales à zéro est atteinte que la surveillance de l'état d'hygiène lors de la production est commutée sur le capteur de dépôt dont la plage de mesure (105) comprend des épaisseurs de couche de dépôt avec une épaisseur de couche de dépôt maximale tolérable prédéterminée.

12. Procédé selon l'une des revendications de procédé précédentes, dans lequel la surveillance du processus de nettoyage initié est d'abord effectuée par le capteur de dépôt dont la plage de mesure inférieure comprend des épaisseurs de couche de dépôt avec une épaisseur de couche de dépôt maximale tolérable prédéfinie, et où ce n'est que lorsque l'extrémité inférieure de la plage de mesure du capteur de dépôt avec une plage de mesure (105) pour des épaisseurs de couche de dépôt ayant une épaisseur de couche de dépôt maximale tolérable prédéfinie (108) est atteinte, que la surveillance du processus de nettoyage initié est commutée sur le capteur de dépôt dont la plage de mesure inférieure comprend des épaisseurs de couche de dépôt proches ou égales à zéro.

13. Procédé selon l'une quelconque des revendications précédentes, dans lequel la surveillance du processus de nettoyage initié comprend en outre la surveillance d'un rinçage, par exemple avec de l'eau, un milieu de nettoyage, la surveillance du rinçage étant effectuée au moyen d'un capteur configuré pour mesurer une propriété physique d'un milieu (308) traversant l'endroit à nettoyer, par exemple configuré pour mesurer la conductivité électrique d'un milieu (308) traversant l'endroit à nettoyer.

14. Procédé selon l'une quelconque des revendications précédentes, dans lequel l'installation de traitement est une installation alimentaire.
